# EUROPEAN PATENT SPECIFICATION

(11) **EP 2 981 223 B1**
(45) Date of publication and mention of the grant of the patent: **27.09.2017**
(21) Application number: 14718464.2
(22) Date of filing: 01.04.2014
(51) Int. Cl.: A61B 18/20, A61B 18/00

(54) **MEDICAL LASER APPARATUS**
MEDIZINISCHE LASERVORRICHTUNG
APPAREIL LASER MÉDICAL

(30) Priority: 01.04.2013 GB 201305881
(43) Date of publication of application: 10.02.2016
(73) Proprietor: Lumenis Ltd., 20692 Yokneam Ilit (IL)
(72) Inventor: IGER, Yoni, Yokneam 2069204 (IL)
(74) Representative: Stütz, Jan
(86) International application number: PCT/IB2014/060352
(87) International publication number: WO 2014/162263

(56) References cited:
- EP-A2- 2 548 617
- US-A- 6 149 645
- US-A1- 2002 095 143
- US-A1- 2007 219 601
- US-A1- 2011 077 627

## Description

### Summary of the Invention

The present invention relates to a medical laser apparatus and a method of operating a medical laser apparatus, cosmetic treatment of skin.

### Background to the Invention

Non-ablative treatment of skin using lasers is an established technique. It has been used for treatment of a number of conditions, including striae, dyschromia and lesions in the skin such as age spots and melasma, and the treatment of wrinkles, acne scars and surgical scars.

In non-ablative treatment, a laser beam at a suitable wavelength and power is directed at a location of the skin, such that the skin surface is not removed or ablated but skin tissue is locally heated. The local heating causes coagulation of the tissue, creating a microscopic, demarcated and isolated, conical thermo-coagulation wound zone. An array of such coagulated zones is created across the skin area to be treated.

The wound coagulation zones cause collagenesis and remodelling of the skin, by releasing and activating different interleukins, heat shock proteins, growth factors and other wound healing mediators. The skin around each thermo-coagulation zone is undamaged, and fibroblasts migrate to the wounded zone and in particular are effective there in synthesis of collagen. The technique is advantageous as the stratum corneum, the top of the skin, remains intact and the skin itself is not ablated, reducing the risk of potential side effects and infection, and lowering patient discomfort.

### Summary of the Invention

According to a first aspect of the invention there is provided a medical apparatus as defined in claim 1.

EP 2 548 617 A2 teaches a similar apparatus, with a broadly defined pulse length from 10 ps to 100 us.

The first laser light beam has a diameter in the range of approximately 100 microns to 400 microns, and preferably in the range 110 to 120 microns.

The second laser light beam may have a diameter in the range of about 2 to 10 mm, and preferably about 2 to 5 mm.

The first wavelength may be in the range 1300 nm to 1600 nm, and preferably about 1565 nm, preferably such that the first laser beam has a desired coagulative effect.

The second wavelength may be in the range 500 to 1300 nm.

The apparatus is operable to generate the second laser light beam with a pulse length in the range 1 to 100 ns, and preferable less than 10 ns, and most preferable 6 to 8 ns.

Also disclosed is an apparatus being operable to generate the second laser light beam with a pulse length in the range 1 to 700 ps.

The apparatus may comprise a treatment head wherein at least the first laser light beam is supplied to the treatment head.

The treatment head may comprise a scanning device, the scanning device being operable to direct the first laser light beam to a plurality of locations within a treatment area.

The first laser source may comprise a fibre laser.

The second laser source may comprise a Q-switched laser.

According to a second aspect of the invention there is provided a method as defined in claim 15.

The first laser light beam has a diameter in the range of approximately 100 microns to 400 microns, and preferably in the range 110 to 120 microns.

The second laser light beam may have a diameter in the range of about 2 to 10 mm, and preferably in the range 2 to 5 mm.

The first wavelength may be in the range 1300 nm to 1600 nm, and preferably about 1565 nm, preferably such that the first laser beam has a desired coagulative effect.

The second wavelength may be in the range 500 to 1300 nm.

The second laser light beam has a pulse length in the range 1 to 100 ns, and preferably less than 10 ns, and most preferably 6 to 8 ns.

The method may comprise directing the first laser light beam to a plurality of locations within a treatment area.

Generating the first laser light beam may comprise operating a fibre laser.

Generating the second laser light beam may comprise operating a Q-switched laser.

### Brief Description of the Drawings

Embodiments of the invention will now be described by way of example only with reference to the accompanying drawings wherein;
Fig. 1a is a diagrammatic illustration of a first apparatus embodying the invention,
Fig. 1b is a diagrammatic illustration of a second apparatus embodying the invention,
Fig. 1c is a diagrammatic illustration of a third apparatus embodying the invention,
Fig. 2 is an illustrative cross-section through a dermis and epidermis during a first irradiation step,
Fig. 3 is a view of a plurality of alternative beam locations of the step of Fig. 2, and
Fig. 4 is a view similar to Fig. 2 during a subsequent irradiation step.

### Detailed Description

With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the invention. In this regard, no attempt is made to show structural details of the invention in more detail than is necessary for a fundamental understanding of the invention, the description taken with the drawings making apparent to those skilled in the art how the several forms of the invention may be embodied in practice.

Before explaining at least one embodiment of the invention in detail, it is to be understood that the invention is not limited in its application to the details of construction and the arrangement of the components set forth in the following description or illustrated n the drawings. The invention is applicable to other embodiments or of being practiced or carried out in various ways. Also, it is to be understood that the phraseology and terminology employed herein is for the purpose of description and should not be regarded as limiting.

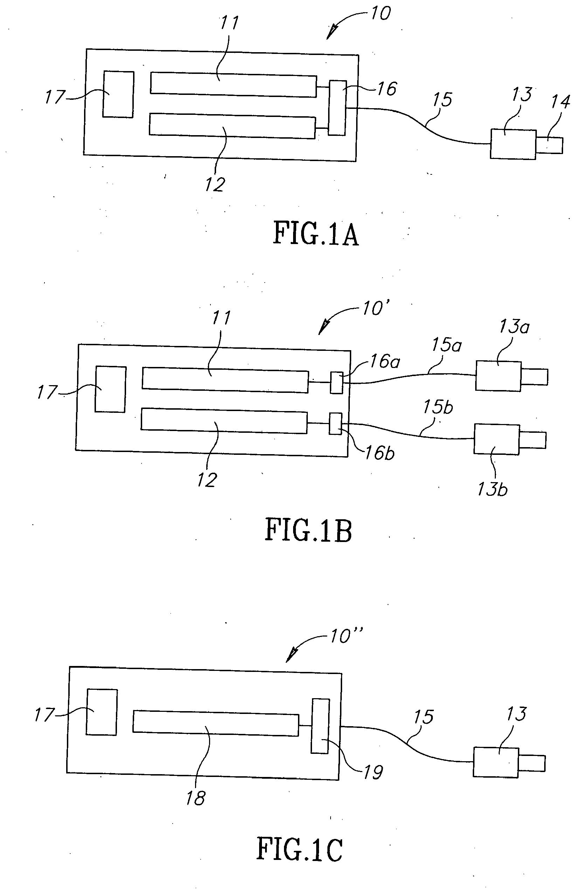

Referring now to Fig. 1a, a medical apparatus is illustrated generally at 10. The apparatus 10 comprises a first laser source 11, and a second laser source 12. In this description, 'laser source' is intended to refer to the laser cavity or resonant element, together with any required control elements and beam conditioning or modulation elements. A treatment head is provided shown at 13, in the present example with an adjustable end part 14. The treatment head 13 is linked to the laser sources by a suitable connection shown at 15, which may be an optical fibre connection, or an internally reflective arm, or any other suitable connection as desired. A beam direction apparatus 16 transmits a first laser beam from the first laser source or a second laser beam from the second laser source 12 to the connection 15 as desired. A controller 17 is provided to control the respective laser sources 11, 12 and provide beam power and characteristics as desired under the control of a suitable operator.

In an alternative configuration, as shown in Fig. 1b, the apparatus 10' may have separate treatment heads 13a, 13b, connected to the first laser source 11, second laser source 12 respectively, by suitable connectors 15a, 15b and optical components 16a, 16b. In a further alternative configuration, apparatus 10" comprises a single laser element 18 and beam modulation optics 19. By controlling the laser element 18 and modulation optics 19, in a first mode of operation the system may operate as a first laser source and generate the first laser beam, and in a second mode of operation operate as a second laser source and generate the second laser beam. The modulation optics 19 may be operable to provide frequency doubling or electro-optical manipulation in any other way to produce a second laser beam which is different than the first laser beam.

The first laser source 11 may have a wavelength in the range 1100 nm to 1600 nm, and in the present example comprises a fibre laser with an output wavelength of 1565 nm and a beam energy of 10 to 70 mJ. The first laser light beam from the first laser source 11 is either a continuous wave beam, or has a relatively long pulse length, of up to 0.5 s. The second laser source 12 comprises a Q-switched laser, in the present example a Q-switched 1064 nm Nd:YAG laser. Q-switching is a known technique in which the energy in a laser cavity is released in a very short, high-power pulse. In this example, the pulse length is in the range 1 to 100 ns, and preferably less than 10 ns and more preferably in the range 6 to 8 ns, although any other appropriate pulse length may be used as desired. If shorter pulse lengths are required, the second laser source 12 could be a picoseconds-laser source, which permits the pulse length to be in the range 1 to 700 ps. Nd:YAG lasers may be operated to obtain a desired wavelength, preferably in the range 500 to 1300 nm. Other laser types, such as an Er:YAG, thulium, holmium or other known solid state, fibre or gas lasers, may alternatively be used.

The adjustable head 14 of the treatment head 13 preferably comprises a scanning device operable to direct the first laser light beam to a plurality of locations over a desired area for treatment. In this example, the first laser light beam preferably has a diameter in the range 100 to 400 microns, and preferably about 110 to 120 microns. The beam is directed to a plurality of locations over a much larger area of skin as needed, for example having a diameter to the range 5 to 20 mm, such that the beam is directed to 50 to 500 spots per cm² of treated skin.

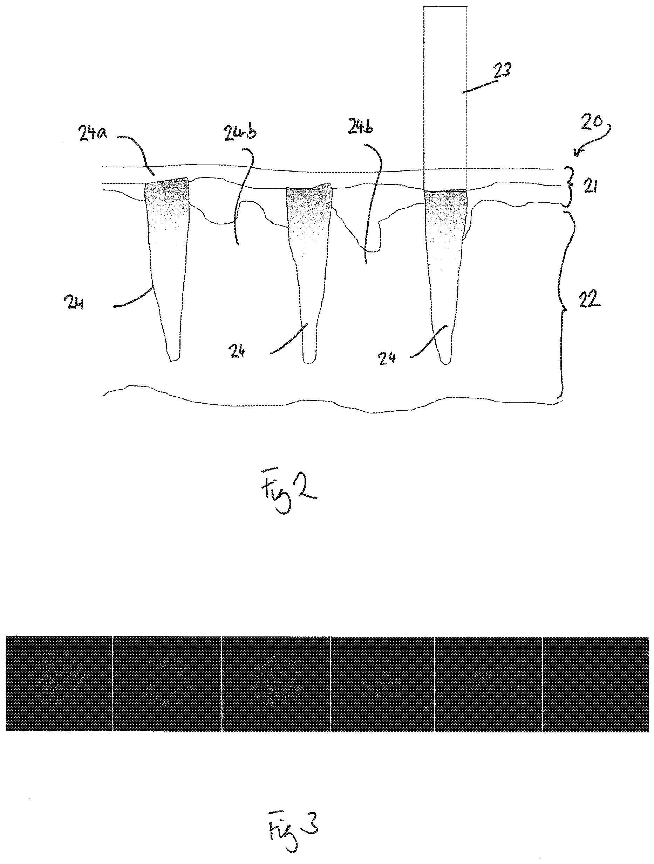

During operation of the first laser source 11, a skin section is generally shown at 20 in Fig. 2. The layers of the epidermis are generally shown at 21 and the dermis illustrated at 22. The first laser light beam is diagrammatically illustrated at 23. As shown at 24, in a first irradiation step the beam 23 is directed at the skin 20, where a local fractional volume of the skin is heated to coagulative level. The wavelength of the first laser beam 23 is selected to have a desired coagulative impact. The treated areas have a width which depends on the width of the first beam 23, in this example generally in the range 100 to 400 microns. The areas 24 extend into the dermis depending on the wavelength and power of the first laser light beam 23 and the length of time the area is irradiated, in this example up to 800 microns. As illustrated at 24a, the upper layer of the epidermis is undamaged or substantially undamaged, reducing the chance of microbial infections. As illustrated at 24b, the regions of skin 20 between the areas 24 are substantially unharmed and unaffected. The first laser light beam 23 may be directed to form an array of areas 24 in any suitable manner as required, depending on the area and condition to be treated. Suitable patterns are illustrated in Fig. 3, including hexagonal, annular, circular, square, rectangular and linear arrangements of areas 24.

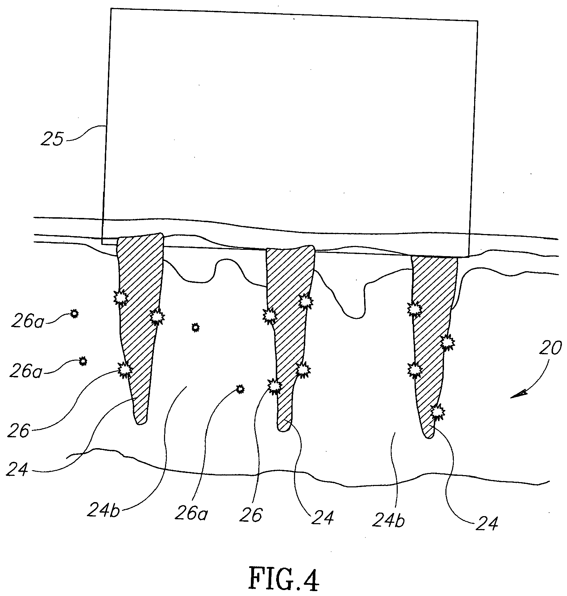

In a subsequent irradiation step, a second laser light beam is generated from the second laser source 12 and directed to the treated area of skin 20 as shown in Fig. 4. In this example, it will be apparent that the second laser light beam 25 has a much larger diameter than the first laser light beam 23, for example, second laser light beam 25 may have a beam width of 1 to 10 mm, preferably 2 to 5 mm (note that the illustration in Fig. 4 is not to scale) and as such will be able to cover a portion of the array of areas 24 illustrated in Fig. 3, thus irradiating a plurality of areas 24 simultaneously. The pulsed second laser light beam 25 affects in particular the coagulated wound tissue of the zones 24 possibly in response to modified elasticity or transparency of the zones 24. The very short laser light pulses cause photomechanical or photoacoustic impacts or shocks illustrated at 26 at the boundaries of the areas 24.The shocks 26 cause localized damage at the boundaries of zones 24b. The second laser light beam may also cause (possibly smaller) shocks in the areas 24b, as illustrated at 26a.The localised damage enhances the release and diffusion of wound healing mediators within and between areas 24 and otherwise unaffected zones 24b, initiating wound healing activity and collagenesis in zones 24b.

Consequently, by subsequently illuminating an area previously treated in a non-ablative manner, with a pulsed laser source, collagen synthesis and skin regeneration over the entire skin area is increased, not simply within or just around the zones 24, leading to an unexpected improvement in the efficacy of the process.

Although in the preceding example, a plurality of zones 24 formed by directing first laser beam 23, and then are subsequently illuminated by second laser beam 25, it will be apparent that this may be performed in any other manner. For example, using the apparatus of Fig. 1a, beam switching device 16 could be operated to direct first laser beam 23 to create an area 24, an then to subsequently direct beam 25 to illuminate the newly created area 24, and then repeat the cycle to create a new area 24 and subsequently re-illuminate the same area. Alternatively, in the example of Fig. 1a, the array of areas 24 may be formed, and then head 14 adjusted or removed to provide a desired width for beam 25. In the example of Fig. 1b, the treatment heads 13a, 13b may be separately adjustable as desired. Further alternatively, the method may be performed using separate apparatuses, one of which has a first laser source 11 and another of which has a second laser source 12.

The apparatus and method described above are suitable for treatment of a range of skin conditions, including the reduction or removal of scars, wrinkles, discoloration and other cosmetic, aesthetic or dermatological issues.

In the above description, an embodiment is an example or implementation of the invention. The various appearances of "one embodiment", "an embodiment" or "some embodiments" do not necessarily all refer to the same embodiments.

Although various features of the invention may be described in the context of a single embodiment, the features may also be provided separately or in any suitable combination. Conversely, although the invention may be described herein in the context of separate embodiments for clarity, the invention may also be implemented in a single embodiment.

Furthermore, it is to be understood that the invention can be carried out or practiced in various ways and that the invention can be implemented in embodiments other than the ones outlined in the description above.

Meanings of technical and scientific terms used herein are to be commonly understood as by one of ordinary skill in the art to which the invention belong, unless otherwise defined.

## Claims

1. A medical apparatus comprising a first and a second laser source producing first and second laser beams, the laser beams being directable to a skin volume; a controller configured to activate the first and second laser sources; the first laser source producing a first pulsed laser beam having a first wavelength, a relatively long pulse duration and a relatively narrow diameter; the second laser source operable producing a second pulsed laser beam having a second wavelength, a relatively short duration and a diameter wider than the diameter from the first laser source, the controller being configured to first cause activation of the first laser source to cause the first laser beam to be directed to a treatment area of the skin volume, the controller being configured to next cause activation of the second laser source to be directed to the treatment area, the first laser light beam having a diameter in the range of 100 microns to 400 microns, the apparatus being operable to generate the second laser light beam with a pulse length in the range 1 to 100 ns.

2. An apparatus according to claim 1, wherein the first laser light beam is non-ablative.

3. An apparatus according to claim 1 wherein the controller is configured to first cause activation of the first laser beam to be directed to a treatment area of the skin volume to form one or more narrow coagulated zones within the skin volume; the controller being configured to next cause activation of the second laser source to be directed to the treatment area to cause photomechanical, photoacoustic or shock effects in the skin volume at least at the boundaries around the one or more narrow coagulated zones created by the first laser beam.

4. An apparatus according to claim 3, wherein the first laser beam is directed to a plurality of locations in a treatment area of the skin volume, and wherein the uncoagulated effects in the skin volume are additionally formed in the areas between the narrow coagulated zones.

5. An apparatus according to claim 1 wherein the first laser light beam has a diameter in the range 110 to 120 microns.

6. An apparatus according to claim 1 wherein the second laser light beam has a diameter in the range of 2 to 10 mm.

7. An apparatus according to claim 5 wherein the second laser light beam has a diameter in the range of 2 to 5 mm.

8. An apparatus according to claim 1 wherein the first wavelength is in the range 1300 to 1600 nm.

9. An apparatus according to claim 7 wherein the first wavelength is 1565nm.

10. An apparatus according to claim 1 wherein the second wavelength is in the range 500 to 1300 nm.

11. An apparatus according to claim 10, wherein the apparatus is operable to generate the second laser light beam with a pulse length of less than 10 ns.

12. An apparatus according to claim 10 wherein the apparatus is operable to generate the second laser light beam with a pulse length of 6 to 8 ns.

13. An apparatus according to claim 1 comprising a treatment head wherein at least the first laser light beam is supplied to the treatment head, and wherein the treatment head comprises a scanning device, the scanning device being operable to direct the first laser light beam to a plurality of locations within a treatment area.

14. An apparatus according to claim 1 wherein the first laser source comprises a fibre laser and wherein the second laser source comprises a Q-switched laser.

15. A method of cosmetically treating a skin surface by directing laser light to a skin volume, the method comprising the steps of providing a first and a second laser source producing first and second laser beams; activating the first and second laser sources wherein the first laser source produces a first pulsed laser light beam having a first wavelength, a relatively long duration and a relatively narrow diameter and second laser source produces a second pulsed laser light beam having a second wavelength, a relatively short pulse duration and a diameter wider than the diameter from the first laser source; wherein the first laser light beam has a diameter in the range of 100 microns to 400 microns and wherein the second laser light beam has a pulse length in the range 1 to 100 ns.

16. A method according to claim 15 wherein the first laser source is activated first to cause the first laser beam to be directed to the treatment area of the skin volume to form one or more narrow coagulated zones within the skin volume; and the second laser source is activated after the first to cause the second laser source to be directed to the treatment area to cause photomechanical, photoacoustic or shock effects in the skin volume at least at the boundaries around the one or more narrow coagulated zones created by the first laser beam.

## Patentansprüche

1. Medizinisches Gerät mit einer ersten und einer zweiten Laserquelle, die erste und zweite Laserstrahlen erzeugen, wobei die Laserstrahlen auf ein Hautvolumen gerichtet werden können; mit einem Controller, der dazu ausgebildet ist, die erste und zweite Laserquelle zu aktivieren; wobei die erste Laserquelle einen ersten gepulsten Laserstrahl mit einer erste Wellenlänge, einer relativ langen Pulsdauer und einem relativ schmalen Durchmesser erzeugt; die zweite LaserQuelle bedienbar ist, einen zweiten gepulsten Laserstrahl mit einer zweiten Wellenlänge, einer relativ kurzen Dauer und einem Durchmesser zu erzeugen, der größer ist, als der Durchmesser von der ersten Laserquelle, wobei der Controller dazu ausgebildet ist, zuerst eine Aktivierung der ersten Laserquelle zu bewirken, um zu bewirken, dass der erste Laserstrahl auf einen Behandlungsbereich des Hautvolumens gerichtet wird, wobei der Controller dazu ausgebildet ist als nächstes die Aktivierung der zweiten Laserquelle zu bewirken, die auf den Behandlungsbereich gerichtet wird, wobei der erste Laserlichtstrahl einen Durchmesser im Bereich von 100 Mikrometern bis 400 Mikrometern besitzt, das Gerät betreibbar ist, den zweiten Laserlichtstrahl mit einer Pulslänge im Bereich von 1 bis 100 ns zu erzeugen.

2. Gerät nach Anspruch 1, **dadurch gekennzeichnet, dass** der erste Laserlichtstrahl nicht abladierend ist.

3. Gerät nach Anspruch 1, **dadurch gekennzeichnet, dass** der Controller dazu ausgebildet ist, zunächst die Aktivierung des ersten Laserstrahls zu bewirken, der auf einen Behandlungsbereich des Hautvolumens gerichtet wird, um einen oder mehr schmale koagulierte Zonen innerhalb des Hautvolumens zu bilden; wobei der Controller dazu ausgebildet ist, als nächstes die Aktivierung der zweiten Laserquelle zu bewirken, die auf den Behandlungsbereich gerichtet wird und photomechanische, photoakustische oder Schock-Effekte im Hautvolumen zumindest an den Grenzen um die ein oder mehrere schmalen Koagulierte Zonen hervorruft, die durch den ersten Laserstrahl erzeugt wurden.

4. Gerät nach Anspruch 3, **dadurch gekennzeichnet, dass** der erste Laserstrahl auf mehrere Stellen in einem Behandlungsbereich des Hautvolumens gerichtet ist und wobei die unkoagulierten Effekte in dem Hautvolumen zusätzlich in den Bereichen zwischen den engen koagulierten Zonen gebildet werden.

5. Gerät nach Anspruch 1, **dadurch gekennzeichnet, dass** der erste Laserlichtstrahl einen Durchmesser in dem Bereich von 110 bis 120 Mikrometer besitzt.

6. Gerät nach Anspruch 1, **dadurch gekennzeichnet, dass** der zweite Laserlichtstrahl einen Durchmesser in dem Bereich von 2 bis 10 mm aufweist.

7. Gerät nach Anspruch 5, **dadurch gekennzeichnet, dass** der zweite Laserlichtstrahl einen Durchmesser in dem Bereich von 2 bis 5 mm aufweist.

8. Gerät nach Anspruch 1, **dadurch gekennzeichnet, dass** die erste Wellenlänge im Bereich von 1300 bis 1600 nm liegt.

9. Gerät nach Anspruch 7, wobei die erste Wellenlänge 1565 nm beträgt.

10. Gerät nach Anspruch 1, wobei die zweite Wellenlänge im Bereich von 500 bis 1300 nm liegt.

11. Gerät nach Anspruch 10, wobei das Gerät dazu ausgebildet ist, den zweiten Laserlichtstrahl mit einer Pulslänge von weniger als 10 ns zu erzeugen.

12. Gerät nach Anspruch 10, **dadurch gekennzeichnet, dass** das Gerät dazu ausgebildet ist, den zweiten Laserlichtstrahl mit einer Pulslänge von 6 bis 8 ns zu erzeugen.

13. Gerät nach Anspruch 1, mit einem Behandlungskopf, wobei zumindest der erste Laserlichtstrahl zu dem Behandlungskopf zugeführt wird und wobei der Behandlungskopf eine Abtastvorrichtung aufweist, wobei die Abtastvorrichtung betreibbar ist, um den ersten Laserlichtstrahl auf eine Vielzahl von Stellen innerhalb eines Behandlungsbereichs zu leiten.

14. Gerät nach einem der Ansprüche 1 bis 13, **dadurch gekennzeichnet, dass** die erste Laserquelle einen Faserlaser umfasst und wobei die zweite Laserquelle einen Q-geschalteten Laser umfasst.

15. Verfahren zur kosmetischen Behandlung einer Hautoberfläche durch Leiten von Laserlicht auf ein Hautvolumen, umfassend die Schritte: Bereitstellen einer ersten und einer zweiten Laserquelle, welche erste und zweite Laserstrahlen erzeugen; Aktivieren der ersten und zweiten Laserquellen, wobei die erste Laserquelle einen ersten gepulsten Laserlichtstrahl mit einer ersten Wellenlänge, einer relativ langen Dauer und einem relativ schmalen Durchmesser erzeugt und die zweite Laserquelle einen zweiten gepulsten Laserlichtstrahl mit einer zweiten Wellenlänge, einer relativ kurzen Pulsdauer und einem Durchmesser erzeugt, der größer ist, als der Durchmesser von der ersten Laserquelle; wobei der erste Laserlichtstrahl einen Durchmesser im Bereich von 100 Mikrometern bis 400 Mikrometern aufweist und wobei der zweite Laserlichtstrahl eine Pulslänge im Bereich von 1 bis 100 ns aufweist.

16. Verfahren nach Anspruch 15, wobei die erste Laserquelle zuerst aktiviert wird, um zu bewirken, dass der erste Laserstrahl auf den Behandlungsbereich des Hautvolumens gerichtet wird, um eine oder mehrere schmal koagulierte Zonen innerhalb des Hautvolumens zu bilden; und die zweite Laserquelle nach der ersten aktiviert wird, um zu bewirken, dass die zweite Laserquelle auf den Behandlungsbereich gerichtet wird, um in dem Hautvolumen zumindest an den Grenzen um die eine oder mehrere schmal koagulierte Zonen, die durch den ersten Laser strahl erzeugt wurden, photomechanische, photoakustische oder Schock-Effekte zu erzeugen.

## Revendications

1. Appareil médical comprenant une première et une seconde source laser produisant un premier et un second faisceau laser, les faisceaux laser pouvant être dirigés sur un volume de peau, un contrôleur configuré pour activer la première et la seconde source laser, la première source laser produisant un premier faisceau laser pulsé qui a une première longueur d'onde, une durée d'impulsion relativement longue et un diamètre relativement étroit, la seconde source laser pouvant être mise en oeuvre produisant un second faisceau laser pulsé qui a une seconde longueur d'onde, une durée relativement courte et un diamètre plus large que le diamètre de la première source laser, le contrôleur étant configuré pour provoquer tout d'abord l'activation de la première source laser pour faire en sorte que le premier faisceau laser soit dirigé vers une zone de traitement du volume de peau, le contrôleur étant configuré pour provoquer ensuite l'activation de la seconde source laser à diriger sur la zone de traitement, le premier faisceau de lumière laser ayant un diamètre de l'ordre de 100 microns à 400 microns, l'appareil pouvant être mis en oeuvre pour générer le second faisceau de lumière laser avec une longueur d'impulsion de l'ordre d'1 à 100 ns.

2. Appareil selon la revendication 1, le premier faisceau de lumière laser étant non ablatif.

3. Appareil selon la revendication 1, le contrôleur étant configuré pour provoquer tout d'abord l'activation du premier faisceau laser à diriger sur une zone de traitement du volume de peau pour former une ou plusieurs zones coagulées étroites à l'intérieur du volume de peau, le contrôleur étant configuré pour provoquer ensuite l'activation de la seconde source laser à diriger sur la zone de traitement pour provoquer des effets photomécaniques, photoacoustiques ou de choc dans le volume de peau au moins sur les limites autour de la ou des plusieurs zones coagulées étroites créées par le premier faisceau laser.

4. Appareil selon la revendication 3, le premier faisceau laser étant dirigé sur une pluralité d'endroits dans une zone de traitement du volume de peau et les effets non coagulés dans le volume de peau sont de plus formés dans les zones entre les zones coagulées étroites.

5. Appareil selon la revendication 1, le premier faisceau de lumière laser ayant un diamètre de l'ordre de 110 à 120 microns.

6. Appareil selon la revendication 1, le second faisceau de lumière laser ayant un diamètre de l'ordre de 2 à 10 mm.

7. Appareil selon la revendication 1, le second faisceau de lumière laser ayant un diamètre de l'ordre de 2 à 5 mm.

8. Appareil selon la revendication 1, la première longueur d'onde étant de l'ordre de 1300 à 1600 nm.

9. Appareil selon la revendication 7, la première longueur d'onde étant de l'ordre de 1565 nm.

10. Appareil selon la revendication 1, la seconde longueur d'onde étant de l'ordre de 500 à 1300 nm.

11. Appareil selon la revendication 10, l'appareil pouvant être mis en oeuvre pour générer le second faisceau de lumière laser avec une longueur d'impulsion de moins de 10 ns.

12. Appareil selon la revendication 10, l'appareil pouvant être mis en oeuvre pour générer le second faisceau de lumière laser avec une longueur d'impulsion de 6 à 8 ns.

13. Appareil selon la revendication 1 comprenant une tête de traitement, au moins le premier faisceau de lumière laser étant délivré sur la tête de traitement et la tête de traitement comprenant un dispositif de balayage, le dispositif de balayage pouvant être mis en oeuvre pour diriger le premier faisceau de lumière laser sur une pluralité d'endroits à l'intérieur d'une zone de traitement.

14. Appareil selon la revendication 1, la première source laser comprenant un laser à fibre et la seconde source laser comprenant un laser déclenché.

15. Procédé de traitement cosmétique d'une surface cutanée en dirigeant une lumière laser sur un volume de peau, le procédé comprenant les étapes de fourniture d'une première et d'une seconde source laser produisant un premier et un second faisceau laser, d'activation de la première et de la seconde source laser, la première source laser produisant un premier faisceau de lumière laser pulsée qui a une première longueur d'onde, une durée relativement longue et un diamètre relativement étroit et la seconde source laser produisant un second faisceau de lumière laser pulsée qui a une seconde longueur d'onde, une durée d'impulsion relativement courte et un diamètre plus large que le diamètre de la première source laser, le premier faisceau de lumière laser ayant un diamètre de l'ordre de 100 microns à 400 microns et le second faisceau de lumière laser ayant une longueur d'impulsion de l'ordre d'1 à 100 ns.

16. Procédé selon la revendication 15, la première source laser étant activée pour tout d'abord faire en sorte que le premier faisceau laser soit dirigé sur la zone de traitement du volume de peau pour former une ou plusieurs zones coagulées étroites à l'intérieur du volume de peau et la seconde source laser étant activée pour tout d'abord faire en sorte que la seconde source laser soit dirigée sur la zone de traitement pour provoquer des effets photomécaniques, photoacoustiques ou de choc dans le volume de peau au moins sur les limites autour de la ou des plusieurs zones coagulées étroites créées par le premier faisceau laser.
